(19) Europäisches Patentamt
European Patent Office
Office européen des brevets

(11) EP 0 983 995 B1

(12) **EUROPÄISCHE PATENTSCHRIFT**

(45) Veröffentlichungstag und Bekanntmachung des Hinweises auf die Patenterteilung:
**31.03.2004 Patentblatt 2004/14**

(21) Anmeldenummer: **99119109.9**

(22) Anmeldetag: **22.02.1997**

(51) Int Cl.[7]: **C07C 217/62**, C07C 215/54, C07C 219/28, C07C 211/28, C07C 211/29, C07C 215/64, C07D 333/58, A61K 31/135, A61K 31/235, A61K 31/38

# (54) Dimethyl-(3-aryl-but-3-enyl)-aminverbindungen als pharmazeutische Wirkstoffe

Pharmaceutically-active dimethyl-(3-aryl-but-3-enyl)-amine compounds

Composés diméthyl-(3-aryl-but-3-ènyl)-amine pharmaceutiquement actifs

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **13.03.1996 DE 19609847**

(43) Veröffentlichungstag der Anmeldung:
**08.03.2000 Patentblatt 2000/10**

(62) Dokumentnummer(n) der früheren Anmeldung(en) nach Art. 76 EPÜ:
**97102923.6 / 0 799 819**

(73) Patentinhaber: **Grünenthal GmbH**
**52078 Aachen (DE)**

(72) Erfinder:
- **Buschmann, Helmut Heinrich, Dr.**
  **52066 Aachen (DE)**
- **Strassburger, Wolfgang Werner Alfred, Prof. Dr.**
  **52146 Würselen (DE)**
- **Koegel, Babette-Yvonne, Dr.**
  **52379 Lanerwehe-Hamisch (DE)**
- **Friedrichs, Elmar Josef, Dr.**
  **52223 Stolberg (DE)**

(56) Entgegenhaltungen:
**EP-A- 0 176 049** **EP-A- 0 693 475**
**US-A- 3 652 589**

- **FLICK K ET AL: "UNTERSUCHUNGEN ZUR CHEMISCHEN STRUKTUR UND ANALGETISCHEN WIRKUNG VON PHENYLSUBSTITUIERTEN AMINOMETHYLCYCLOHEXANOLEN" ARZNEIMITTEL FORSCHUNG. DRUG RESEARCH, Bd. 28, Nr. 1A, 1. Januar 1978 (1978-01-01), Seiten 107-113, XP000608150**
- **KATO, TAKESHI ET AL: "Synthesis and analgesic activity of cyclohexenylmethylamines and related compounds" CHEM. PHARM. BULL. (1984), 32(6), 2279-89, XP002031715**
- **CHEMICAL ABSTRACTS, vol. 54, no. 20, 25. Oktober 1960 (1960-10-25) Columbus, Ohio, US; abstract no. 20963c, I. N. NAZAROV ET AL.: "Synthetic analgesic substances. XXX. Synthesis and study of p-methoxy- and p-chlorophenyldimethylaminopropanols and their esters" XP002031716 & IZVEST. AKAD. NAUK. S.S.S.R., OTDEL. KHIM. NAUK., 1960, Seiten 251-8,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 983 995 B1

## Beschreibung

Die Erfindung betrifft Dimethyl-(3-aryl-but-3-enyl)-aminverbindungen, Verfahren zu deren Herstellung sowie die Verwendung dieser Verbindungen in Arzneimitteln.

Die Behandlung chronischer und nichtchronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik sowie der Grundlagenforschung zur Nociception in letzter Zeit erschienen sind.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation, hervorrufen. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990).

Tramadolhydrochlorid - (1 RS, 2RS)-2-Dimethylaminomethyl-1-(3-methoxyphenyl)-cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267, 331 (1993)). Tramadol ist ein Racemat und besteht aus gleichen Mengen an (+)- und (-)-Enantiomer. In vivo bildet der Wirkstoff den Metaboliten O-Desmethyl-tramadol, der gleichfalls als Enantiomerengemisch vorliegt. Untersuchungen haben ergeben, daß sowohl die Enantiomeren von Tramadol als auch die Enantiomeren der Tramadolmetabolite an der analgetischen Wirkung beteiligt sind (J. Pharmacol. Exptl. Ther. 260, 275 (1992)).

Die EP 0 176 049 A2 des "Consiglio Nazionale delle Richerche" beschreibt pharmakologisch aktive Aminoalkyl-naphthalen-Derivate.

Die EP 693 475 A1 der Grünenthal GmbH beschreibt pharmakologisch aktive 1-Phenyl-3-dimethylaminopropanverbindungen.

In der Arbeit "Untersuchungen zur chemischen Struktur und analgetischen Wirkung von phenylsubstituierten Aminomethylcyclohexanolen" von K.

Flick et al., Arzneim,. Forsch. 28 (I), Heft 1a (1978) wurde die analgetische Wirksamkeit verschiedener phenylsubstituierter Aminomethylcyclohexanole untersucht. Dabei wurde auf Struktur-Wirkungsbeziehungen eingegangen und der Quotient aus akuter Toxizität und analgetischer Wirksamkeit als Auswahlkriterium für analgetisch interessante Verbindungen angewandt.

Die der Erfindung zugrundeliegende Aufgabe bestand in der Entwicklung von analgetisch wirksamen Substanzen, die sich zur Behandlung starker Schmerzen eignen, ohne die für Opioide typischen Nebenwirkungen hervorzurufen. Darüber hinaus sollten die zu entwickelnden Substanzen nicht die während der Behandlung mit Tramadol in manchen Fällen auftretenden Nebenwirkungen, beispielsweise Übelkeit und Erbrechen, besitzen.

Es wurde gefunden, daß die an die zu entwickelnden Substanzen gestellten Anforderungen von bestimmten Dimethyl-(3-aryl-but-3-enyl)-aminen erfüllt werden. Diese Substanzen zeichnen sich durch eine ausgeprägte analgetische Wirkung aus, die im Vergleich zu Tramadol deutlich verstärkt ist.

Gegenstand der Erfindung sind dementsprechend Dimethyl-(3-aryl-but-3-enyl)-aminverbindungen der Formel I,

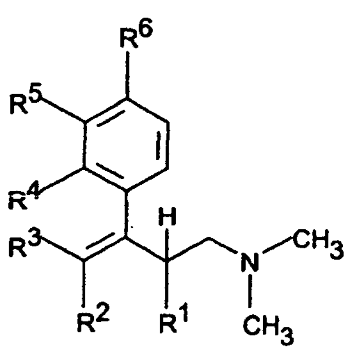

in der $R^1$ $C_{1-5}$-Alkyl ist und $R^2$ H oder $C_{1-5}$-Alkyl bedeutet, $R^3$ H oder $C_{1-5}$-Alkyl bedeutet, $R^4$ H, OH, $C_{1-4}$-Alkyl, O-$C_{1-4}$-Alkyl, O-Benzyl, $CF_3$, O-$CF_3$, Cl, F oder $OR^8$ bedeutet, $R^5$ H, OH, $C_{1-4}$-Alkyl, O-$C_{1-4}$-Alkyl, O-Benzyl, $CHF_2$, $CF_3$,

$O-CF_3$, Cl, F oder $OR^8$ darstellt und $R^6$ H, OH, $C_{1-4}$-Alkyl, O-$C_{1-4}$-Alkyl, O-Benzyl, $CF_3$, $O-CF_3$, Cl, F oder $OR^8$ bedeutet, mit der Maßgabe, daß zwei der Reste $R^4$, $R^5$ oder $R^6$ H sind, oder $R^4$ und $R^5$ zusammen $-CH=C(R^9)-O-$ oder $-CH=C(R^9)-S-$ bedeuten mit der Maßgabe, daß $R^6$ H ist, oder $R^5$ und $R^6$ zusammen $-CH=CH-C(OR^{10})=CH$bedeuten mit der Maßgabe, daß $R^4$ H ist, $R^8$ CO-$C_{1-5}$-Alkyl, PO(O-$C_{1-4}$-Alkyl)$_2$, CO-$C_6H_4$-$R^{11}$, CO(O-$C_{1-5}$-Alkyl), CO-$CHR^{12}$-$NHR^{13}$, CO-NH-$C_6H_3$-$(R^{14})_2$ oder eine Pyridyl-, Thienyl-, Thiazoyl- oder Phenylgruppe bedeutet, $R^9$ H oder $C_{1-4}$-Alkyl bedeutet, $R^{10}$ H oder $C_{1-3}$-Alkyl bedeutet, $R^{11}$ OC(O)-$C_{1-3}$-Alkyl in ortho-Stellung oder $CH_2$-N-$(R^{15})_2$ in meta- oder para-Stellung wobei $R^{15}C_{1-4}$-Alkyl oder beide Reste $R^{15}$ zusammen mit N den 4-Morpholino-Rest bilden, bedeutet, $R^{12}$ und $R^{13}$ gleich oder verschieden sind und H, $C_{1-6}$-Alkyl oder $C_{3-8}$-Cycloalkyl oder $R^{12}$ und $R^{13}$ zusammen $-(CH_2)_{3-8}-$ bedeuten, $R^{14}$ H, OH, $C_{1-7}$-Alkyl, O-$C_{1-7}$-Alkyl, Phenyl, O-Aryl, $CF_3$, Cl oder F bedeutet, mit der Maßgabe, daß die beiden Reste $R^{14}$ gleich oder verschieden sind, in Form ihrer Basen und/oder Salze von physiologisch verträglichen Säuren, als Enantiomere oder Racemate.

Bevorzugte Dimethyl-(3-aryl-3-but-3-enyl)-aminverbindungen entsprechen der Formel I mit $R^1$ $C_{1-3}$-Alkyl und $R^2$ H oder $C_{1-3}$-Alkyl, $R^3$ H oder $C_{1-3}$-Alkyl, $R^4$ H, OH, $CF_3$, Cl, F oder $OR^8$, $R^5$ H, OH, $C_{1-4}$-Alkyl, O-$C_{1-4}$-Alkyl, O-Benzyl, $CHF_2$, $CF_3$, Cl, F oder $OR^8$ und $R^6$ H, OH, O-$C_{1-4}$-Alkyl, O-Benzyl, $CF_3$, Cl, F oder $OR^8$, mit der Maßgabe, daß zwei der Reste $R^4$, $R^5$ oder $R^6$ H sind, oder $R^4$ und $R^5$ zusammen $-CH=C(R^9)-O-$ oder $-CH=C(R^9)-S-$, mit der Maßgabe, daß $R^6$ H ist, oder $R^5$ und $R^6$ zusammen - $CH=CH-C(OR^{10})=CH-$, mit der Maßgabe, daß $R^4$ H ist. Besonders eignen sich Dimethyl-(3-aryl-but-3-enyl)-aminverbindungen der Formel I, in der $R^1$ $CH_3$ oder $C_3H_7$ und $R^2$ H, $CH_3$ oder $CH_2CH_3$ darstellen, $R^3$ H, $CH_3$ oder $CH_2CH_3$ bedeutet, $R^4$ H oder OH, $R^5$ H, OH, $OCH_3$, $CHF_2$ oder $OR^8$ und $R^6$ H, OH oder $CF_3$ bedeuten, mit der Maßgabe, daß zwei der Reste $R^4$, $R^5$ oder $R^6$ H sind, oder $R^4$ und $R^5$ zusammen $-CH=C(CH_3)-S-$ darstellen, mit der Maßgabe, daß $R^6$ H ist, oder $R^5$ und $R^6$ zusammen - CH=CH-C(OH)=CH- bedeuten, mit der Maßgabe, daß $R^4$ H ist, und $R^8$ CO-$C_6H_4$-$R^{11}$ mit $R^{11}$ OC(O)-$C_{1-3}$-Alkyl in ortho-Stellung darstellt. Insbesondere bevorzugt werden Dimethyl-(3-aryl-but-3-enyl)-aminverbindungen mit $R^1$ $CH_3$ und $R^2$ H oder $CH_3$, $R^3$ H oder $CH_3$, $R^4$ H, $R^5$ OH oder $OR^8$, $R^6$ H und $R^8$ CO-$C_6H_4$-$R^{11}$ mit $R^{11}$ OC(O)$CH_3$ in ortho-Stellung.

Weiterer Erfindungsgegenstand ist ein Verfahren zur Herstellung von Dimethyl-(3-aryl-but-3-enyl)-aminverbindungen der Formel I, in der $R^1$ $C_{1-5}$-Alkyl ist und $R^2$ H oder $C_{1-5}$-Alkyl bedeutet, $R^3$ H oder $C_{1-5}$-Alkyl bedeutet, $R^4$ H, $C_{1-4}$-Alkyl, O-$C_{1-4}$-Alkyl, O-Benzyl, $CF_3$, $O-CF_3$, Cl oder F bedeutet, $R^5$ H, $C_{1-4}$-Alkyl, O-$C_{1-4}$-Alkyl, O-Benzyl, $CHF_2$, $CF_3$, $O-CF_3$, Cl oder F darstellt und $R^6$ H, $C_{1-4}$-Alkyl, O-$C_{1-4}$-Alkyl, O-Benzyl, $CF_3$, $O-CF_3$, Cl oder F bedeutet, mit der Maßgabe, daß zwei der Reste $R^4$, $R^5$ oder $R^6$ H sind, oder $R^4$ und $R^5$ zusammen $-CH=C(R^9)-O-$ oder - $CH=C(R^9)-S-$ bedeuten mit der Maßgabe, daß $R^6$ H ist, oder $R^5$ und $R^6$ zusammen $-CH=CH-C(OR^{10})=CH-$ bedeuten, mit der Maßgabe, daß $R^4$ H ist, $R^9$ H oder $C_{1-4}$-Alkyl und $R^{10}$ H oder $C_{1-3}$-Alkyl bedeuten, welches dadurch gekennzeichnet ist, daß man ein β-Dimethylaminoketon der Formel II

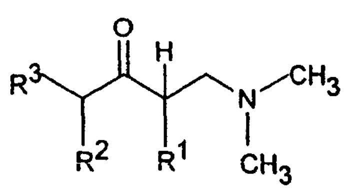

mit einer metallorganischen Verbindung der Formel III

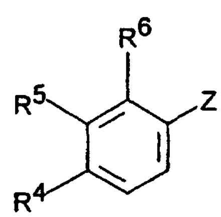

in der Z MgCl, MgBr, MgI oder Li bedeutet, zu einem tertiären Alkohol der Formel IV

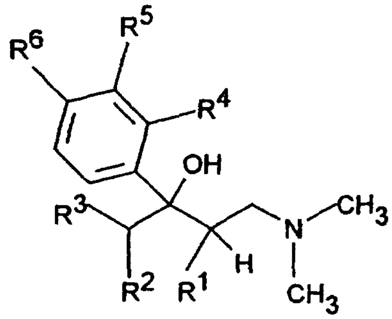

umsetzt, welcher anschließend zu einer Verbindung der Formel I dehydratisiert wird.

Die Reaktion eines β-Dimethylaminoketons mit einer Grignard-Verbindung der Formel III, in der Z MgCl, MgBr oder MgJ bedeutet, oder mit einer lithiumorganischen Verbindung der Formel III kann in einem aliphatischen Ether, beispielsweise Diethylether und/oder Tetrahydrofuran, bei Temperaturen zwischen -70° und +60° C durchgeführt werden. Die Umsetzung mit einer Grignard-Verbindung kann mit oder ohne Zusatz eines Mitführreagenzes, vorzugsweise 1,2-Dibromethan, erfolgen. Lithiumorganische Verbindungen der Formel III lassen sich durch Umsetzung einer Verbindung der Formel III, in der Z Cl, Br oder J bedeutet, mit beispielsweise einer n-Butyllithium/Hexan-Lösung durch Halogen/Lithiumaustausch erhalten.

Die erhaltenen tertiären Alkohole der Formel IV lassen sich mit Säuren, insbesondere Ameisensäure oder Salzsäure, bei Temperaturen zwischen 0 und 100 °C dehydratisieren.

Weiterer Erfindungsgegenstand ist ein Verfahren zur Herstellung einer Dimethyl-(3-aryl-but-3-enyl)-aminverbindung der Formel I, in der $R^1$ $C_{1-5}$-Alkyl ist und $R^2$ H oder $C_{1-5}$-Alkyl bedeutet, $R^3$ H oder $C_{1-5}$-Alkyl bedeutet, einer der Reste $R^4$, $R^5$ oder $R^6$ OH bedeutet und die anderen beiden Reste H sind, welches dadurch gekennzeichnet ist, daß man eine Verbindung der Formel I, in der einer der Reste $R^4$, $R^5$ oder $R^6$ O-$CH_3$ bedeutet und die anderen beiden Reste H sind, mit Diisobutylaluminiumhydrid umsetzt oder eine Verbindung der Formel I, in der einer der Reste $R^4$, $R^5$ oder $R^6$ O-Benzyl bedeutet und die anderen beiden Reste H sind, reduktiv debenzyliert.

Die Umsetzung einer Dimethyl-(3-aryl-but-3-enyl)-aminverbindung mit Diisobutylaluminiumhydrid wird üblicherweise in einem aromatischen Kohlenwasserstoff, beispielsweise Toluol, bei einer Temperatur zwischen 60 und 130 °C durchgeführt (Synthesis 1975, 617; DE 24 09 990, DE 24 09 991; Chem. Abstr. 84, 59862 (1974)).
Die reduktive Debenzylierung einer erfindungsgemäßen Verbindung der Formel I, in der einer der Reste $R^4$, $R^5$ oder $R^6$ O-Benzyl bedeutet, läßt sich in Gegenwart von Platin oder Paladium auf einem Trägermaterial, beispielsweise Aktivkohle, in Gegenwart von Wasserstoff in einem Lösungsmittel, beispielsweise Essigsäure oder $C_{1-4}$-Alkylalkohol, bei Drücken zwischen 1 und 100 bar und Temperaturen zwischen 20 und 100 °C durchführen.

Dimethyl-(3-aryl-but-3-enyl)-aminverbindungen der allgemeinen Formel I, in der einer oder mehrere der Aromatensubstituenten $R^4$, $R^5$ und $R^6$ $OR^8$ bedeuten und $OR^8$ eine Phosphat-, Carbonat-, Carbamat-, Carboxylatoder Aryloxy- oder Heteroaryloxygruppe darstellt, lassen sich durch Umsetzung einer entsprechenden Dimethyl-[3-(hydroxy-phenyl)-but-3-enyl]-aminverbindung der Formel I, in der $R^4$, $R^5$ und/oder $R^6$ eine OH-Gruppe bedeuten, in Form eines Alkalisalzes mit einem Dialkylchlorophosphat, mit einem Alkylchloroformiat, mit einem Aryl- oder Heteroarylisocyanat, mit einem Carbonsäurechlorid oder einem Aryl- oder Heteroarylhalogenid erhalten. Diese Umsetzungen werden üblicherweise in einem Lösungsmittel, beispielsweise Toluol, Dichlormethan, Diethylether und/oder Tetrahydrofuran bei Temperaturen zwischen -15 und +110 °C durchgeführt (Drugs of the Future 16, 443 (1991); J. Med. Chem. 30, 2008 (1989) und 32, 2503 (1989); J. Org. Chem. 43, 4797 (1978); Tetrahedron Lett. 1977, 1571; J. Pharm. Sci. 57, 774 (1968)). Die Umsetzungen mit einem Aryl- oder Heteroarylhalogenid werden unter Zusatz von Kupferpulver und/oder einem Kupfer-I-halogenid als Katalysator durchgeführt.

Dimethyl-(3-aryl-but-3-enyl)-aminverbindungen der Formel I, in der $OR^8$ eine α-Aminocarboxylatgruppe darstellt, sind durch Umsetzung einer entsprechenden Dimethyl-[3-(hydroxy-phenyl)-but-3-enyl]-aminverbindung der Formel I, in der $R^4$, $R^5$ und/oder $R^6$ eine OH-Gruppe bedeuten, mit einer entsprechenden 2-t-Butoxycarbonylamino-carbonsäure unter Verwendung von Triethylamin und Kupplungsreagenzien, wie Benzotriazol-1-yl-oxy-tripyrrolidinophosphoniumhexafluorophosphat in einem Lösungsmittel, beispielsweise Dichlormethan, erhältlich.

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

Die erfindungsgemäßen Verbindungen haben eine ausgeprägte analgetische Wirkung und sind toxikologisch unbedenklich. Sie eignen sich daher als pharmazeutische Wirkstoffe. Dementsprechend ist Erfindungsgegenstand auch die Verwendung einer Dimethyl-(3-aryl-but-3-enyl)-aminverbindung der Formel I als Wirkstoff in Arzneimitteln, vorzugsweise als Wirkstoff in Schmerzmitteln.

Erfindungsgemäße Arzneimittel enthalten neben mindestens einer Dimethyl-(3-aryl-but-3-enyl)-aminverbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder örtlich, zum Beispiel auf Infektionen an der Haut, an den Schleimhäuten oder an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot in gelöster Form oder in einem Pflaster, gegebenenfalls unter Zusatz von die Hauptpenetration fördernden Mitteln, sind Beispiele für geeignete perkutane Zubereitungsformen. Aus oral oder perkutan anwendbaren Zubereitungsformen können die erfindungsgemäßen Verbindungen verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 10 bis 500 mg pro kg wenigstens einer Dimethyl-(3-aryl-but-3-enyl)-aminverbindung der Formel I appliziert.

**Beispiele**

**<u>Herstellung erfindungsgemäßer Verbindungen</u>**

Die Angabe Ether bedeutet Diethylether.

Als stationäre Phase für die Säulenchromatographie wurde Kieselgel 60 (0,040 - 0,063 mmm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Racemattrennungen wurden auf einer Chiracel OD Säule der Firma Daicel Chemical Industries, LTD durchgeführt.

Die Mischungsverhältnisse der Laufmittel für alle chromatographischen Untersuchungen sind in VolumenNolumen angegeben.

**Beispiel 1**

(Z)-(RS)-[3-(3-Methoxy-phenyl)-2-methyl-pent-3-enyl]-dimethylamin, Hydrochlorid (1)

1. Stufe

(2RS, 3RS)-1-Dimethylamino-3-(3-methoxy-phenyl)-2-methyl-pentan-3-ol, Hydrochlorid (2)

27,0 g (1,11 mol) Magnesiumspäne wurden in 150 ml Tetrahydrofuran gerührt und 207,6 g (1,11 mol) 1-Brom-3-methoxy-benzol, gelöst in 400 ml Tetrahydrofuran, zugetropft. Es wurde eine Stunde unter Rückfluß erhitzt und anschließend auf eine Temperatur zwischen 5 und 10 °C abgekühlt. Bei dieser Temperatur wurden 128,30 g (0,89 mol) (RS)-1-Dimethylamino-2-methyl-pentan-3-on, gelöst in 400 ml Tetrahydrofuran, zugetropft. Das Reaktionsgemisch wurde stehen gelassen und anschließend erneut auf eine Temperatur zwischen 5 und 10 °C abgekühlt. Nach Zugabe von 300 ml 20 gew.%iger Ammoniumchloridlösung wurde mit 400 ml Ether verdünnt. Nach Phasentrennung wurde zweimal mit Ether extrahiert, über Natriumsulfat getrocknet und das Lösungsmittel destillativ entfernt. Der erhaltene Rückstand wurde in 3,2 l 2-Butanon aufgenommen und mit 120,60 g (1,11 mol) Trimethylchlorsilan und 20 ml Wasser versetzt. Es wurden 121,5 g Hydrochlorid (2) (38 % der Theorie) mit einem Schmelzpunkt von 198-199 °C erhalten.

2. Stufe:

(Z)-(RS)-[3-(3-Methoxy-phenyl)-2-methyl-pent-3-enyl]-dimethylamin, Hydrochlorid (1)

200 g (0,69 mol) Hydrochlorid (2) wurden in einem Liter konzentrierter Salzsäure gelöst und bei Raumtemperatur stehengelassen. Die Salzsäure wurde im Vakuum destillativ entfernt. Der Rückstand wurde in 1 l Eiswasser

gelöst und mit 10 molarer Natronlauge ein pH-Wert von 13 eingestellt. Nach Extraktion mit Ether, Trocknen der organischen Phase und destillativer Entfernung des Lösungsmittels wurden 162 g Rohprodukt erhalten, das durch umkristallisieren gereinigt wurde. Es wurden 79 g (42 % der Theorie) Hydrochlorid (1) mit einem Schmelzpunkt von 169-170 °C erhalten.

**Beispiel 2**

(Z)-(RS)-3-[1-(2-Dimethylamino-1-methyl-ethyl)-propenyl]-phenol, Hydrochlorid (3)

Zu 1,6 l 20-gew.%iger Diisobutylaluminiumhydrid-Lösung in Toluol wurden bei Raumtemperatur 182 g (Z)-(RS)-[3-(3-Methoxy-phenyl)-2-methyl-pent-3-enyl]-dimethylamin, gelöst in 360 ml Toluol, getropft. Anschließend wurde 11 Stunden unter Rückfluß erhitzt. Nach Abkühlen auf 0 °C wurden 450 ml Ethanol unter Kühlung zugetropft. Anschließend wurde 15 Minuten gerührt und mit 1 l Toluol verdünnt. Danach wurden 450 ml eines Ethanol/Wasser-Gemisches (1 : 1) unter Kühlung zugetropft. Nach einstündigem Rühren bei Raumtemperatur wurde das ausgefallene Aluminiumhydroxid abgesaugt und aus der organischen Phase Lösungsmittel destillativ entfernt. Es wurden 167 g (97,6 % der Theorie) Rohbase erhalten, die in 1,67 l Aceton gelöst und mit 65 ml konzentrierter Salzsäure versetzt wurde. Es kristallisierten 152 g (76 % der Theorie) Hydrochlorid (3) mit einem Schmelzpunkt von 161- 62 °C aus.

**Beispiel 3**

Enantiomere von (3):

(+)-(Z)-(S)-3-[1-(2-Dimethylamino-1-methyl-ethyl)-propenyl]-phenol, Hydrochlorid (+3) und

(-)-(Z)-(R)-3-[1-(2-Dimethylamino-1-methyl-ethyl)-propenyl]-phenol, Hydrochlorid (-3)

Aus dem nach Beispiel 2 erhaltenen Hydrochlorid (3) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 166-167 °C isoliert.

(+3): Ausbeute: 42 % der Theorie $[\alpha]_D^{RT}$ = +3,6° (c = 1,04; Methanol)

(-3): Ausbeute: 44 % der Theorie $[\alpha]_D^{RT}$ = -3,6° (c = 1,04; Methanol)

**Beispiel 4**

(Z)-(RS)-2-Acetoxy-benzoesäure-3-[1-(2-dimethylamino-1-methyl-ethyl)-propenyl]-phenylester, Hydrochlorid (4)

Aus dem Hydrochlorid (3), hergestellt nach Beispiel 2, wurde mit Dichlormethan/wäßriger Natriumhydrogencarbonat-Lösung die Base freigesetzt und nach dem Trocknen der Lösung das Dichlormethan destillativ entfernt. 0,67 g (3,0 mmol) der erhaltenen Base wurden in 7 ml trockenem Dichlormethan gelöst und bei Raumtemperatur mit 0,6 g (3,24 mmol) 2-Acetyl-benzoyl-chlorid, gelöst in 3 ml trockenem Dichlormethan, versetzt. Nach 20-stündigem Rühren bei Raumtemperatur wurde das Reaktionsgemisch mit 20 ml Natriumhydrogencarbonatlösung versetzt und die wäßrige Phase zweimal mit 10 ml Dichlormethan extrahiert. Die organischen Phasen wurden vereinigt und über Natriumsulfat getrocknet. Nach destillativer Entfernung des Lösungsmittels wurden 1,1 g Rohgemisch gewonnen, das auf eine Säule, gefüllt mit Kieselgel, gegeben wurde. Die Elution mit Ether ergab 0,68 g Base, aus der mit Trimethylchlorsilan/Wasser in Ether 0,68 g (54 % der Theorie) Hydrochlorid (4) mit einem Schmelzpunkt von 86-88 °C erhalten wurden.

**Beispiel 5**

(E)-(RS)-[3-(3-Methoxy-phenyl)-2-methyl-pent-3-enyl]-dimethylamin, Hydrochlorid (5)

75 g (0,26 mol) (2RS,3RS)-1-Dimethylamino-3-(3-methoxy-phenyl)-2-methyl-pentan-3-ol, Hydrochlorid (1) aus Beispiel 1 (Stufe 1) wurden in einem Liter konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/Ether versetzt. Nach Trocknung der organischen Phase und destillativer Entfernung des Lösungsmittels wurden 60 g (98 % der Theorie) Rohbase erhalten ((Z)-Isomer (2): (E)-Isomer (5) = 6 : 4). Die

Rohbase wurde auf eine Säule, gefüllt mit Kieselgel, gegeben. Die Elution mit Diisopropylether/ Methanol = 7 : 1 ergab 20 g Base, aus der mit Trimethylchlorsilan/Wasser in 2-Butanon 18,4 g (26 % der Theorie) Hydrochlorid (5) mit einem Schmelzpunkt von 139-140°C erhalten wurden.

**Beispiel 6**

(E)-(RS)-3-[1-(2-Dimethylamino-1-methyl-ethyl)-propenyl]-phenol, Hydrochlorid (6)

Aus (5), hergestellt nach Beispiel 5, wurde mit Dichlormethan/Natronlauge die Base freigesetzt und nach dem Trocknen der Lösung Dichlormethan destillativ entfernt. Aus der erhaltenen Base wurde unter den in Beispiel 2 angegebenen Bedingungen das Hydrochlorid (6) in einer Ausbeute von 73 % der Theorie und einem Schmelzpunkt von 80 °C erhalten.

**Beispiel 7**

Enantiomere von (6):

(+)-(E)-(R)-3-[1-(2-Dimethylamino-1-methyl-ethyl)-propenyl]-phenol, Hydrochlorid (+6) und

(-)-(E)-(S)-3-[1-(2-Dimethylamino-1-methyl-ethyl)-propenyl]-phenol, Hydrochlorid (-6)

Aus nach Beispiel 6 erhaltenem Hydrochlorid (6) wurde mit Dichlormethan/wäßriger Natriumhydrogencarbonatlösung die Base freigesetzt. Nach dem Trocknen der Lösung wurde Dichlormethan im Vakuum abdestilliert. Das Racemat wurde dann auf einer chiralen HPLC-Säule getrennt. Aus den erhaltenen Enantiomeren wurden durch Umsetzung mit konzentrierter Salzsäure in Aceton die Hydrochloride mit einem Schmelzpunkt von 154-155 °C isoliert.

(+6): Ausbeute: 42 % der Theorie $[\alpha]_D^{RT}$ = +36,3° (c = 0,96; Methanol)

(-6): Ausbeute: 44 % der Theorie $[\alpha]_D^{RT}$ = -33,7° (c = 1,07; Methanol)

**Beispiel 8**

(Z)-(RS)-4-[1-(2-Dimethylamino-1-methyl-ethyl)-propenyl]-phenol, Hydrochlorid (7)

1. Stufe:

(Z)-(RS)-[3-(4-Methoxy-phenyl)-2-methyl-pent-3-enyl]-dimethylamin (8) Ausgehend von (RS)-1-Dimethylamino-2-methyl-pentan-3-on und 1-Brom-4-methoxy-benzol wurde unter den in Beispiel 1 (1. Stufe) angegebenen Bedingungen (2RS, 3RS)-1-Dimethylamino-3-(4-methoxy-phenyl)-2-methyl-pentan-3-ol, Hydrochlorid in einer Ausbeute von 44 % und einem Schmelzpunkt von 188-189 °C erhalten, das unter den in Beispiel 1 (2. Stufe) angegebenen Bedingungen mit konzentrierter Salzsäure in (Z)-(RS)-[3-(4-Methoxy-phenyl)-2-methyl-pent-3-enyl]-dimethylamin (8) überführt wurde. Verbindung (8) wurde als hellgelbes Öl in einer Ausbeute von 46 % erhalten.

2. Stufe:

(Z)-(RS)-4-[1-(2-Dimethylamino-1-methyl-ethyl)-propenyl]-phenol, Hydrochlorid (7)

Aus der nach Stufe 1 erhaltenen Base wurde unter den in Beispiel 2 angegebenen Bedingungen das Hydrochlorid (7) in einer Ausbeute von 79 % der Theorie und einem Schmelzpunkt von 203 °C erhalten.

**Beispiel 9**

(Z)-(RS)-Dimethyl-(2-methyl-3-m-tolyl-pent-3-enyl)amin, Hydrochlorid (9)

Ausgehend von (RS)-1-Dimethylamino-2-methyl-pentan-3-on und 3-Bromtoluol wurde unter den in Beispiel 1 (1. Stufe) angegebenen Bedingungen (2RS, 3RS)-1-Dimethylamin-2-methyl-3-(m-tolyl)-pentan-3-ol, Hydrochlorid in einer Ausbeute von 24 % und einem Schmelzpunkt von 154-155 °C erhalten, das unter den in Beispiel 1 (2. Stufe) angegebenen Bedingungen mit konzentrierter Salzsäure in (Z)-(RS)-Dimethyl-(2-methyl-3-m-tolyl-pent- -3-enyl)-amin,

Hydrochlorid (9) überführt wurde. Verbindung (9) wurde in einer Ausbeute von 36 % (bezogen auf den eingesetzten Alkohol) mit einem Schmelzpunkt von 172 °C erhalten.

**Beispiel 10**

(E)-(RS)-Dimethyl-(2-methyl-3-m-tolyl-pent-3-enyl)amin, Hydrochlorid (10)

Ausgehend von (2RS, 3RS)-1-Dimethylamino-2-methyl-3(m-tolyl)-pentan-3-ol, Hydrochlorid, welches nach Beispiel 9 hergestellt wurde, wurde unter den in Beispiel 5 angegebenen Bedingungen das Hydrochlorid (10) in einer Ausbeute von 36 % mit einem Schmelzpunkt von 153 °C erhalten.

**Beispiel 11**

(Z)-(RS)-[3-(3-Difluoromethyl-phenyl)-2-methyl-pent-3-enyl]-dimethylamin, Hydrochlorid (11)

1. Stufe:

(2RS,3RS)-3-(3-Difluormethyl-phenyl)-1-dimethylamino-2-methyl-pentan-3-ol, Hydrochlorid (12)

7,0 g (34 mmol) 1-Bromo-3-difluormethyl-benzol, hergestellt aus 3-Brombenzaldehyd und Diethylaminoschwefeltrifluorid gemäß Org. React. 35, 513 (1988), wurden in 110 ml trockenem Tetrahydrofuran gelöst und auf -75 °C gekühlt. Nach Zugabe von 34 mmol 1,6-molarer n-Butyllithiumlösung in Hexan wurde eine Stunde bei -75 °C gerührt. Anschließend wurden 4,8 g (34 mmol) (2RS)-1-Dimethylamino-2-methylpentan-3-on, gelöst in 15 ml trockenem Tetrahydrofuran, zugetropft. Innerhalb von 2,5 Stunden wurde das Reaktionsgemisch auf Raumtemperatur erwärmt.

Zur Aufarbeitung wurden unter Eisbadkühlung 65 ml 5 %-ige Salzsäure zugetropft, so daß die Innentemperatur 15 °C nicht überstieg. Nach Phasentrennung wurde die organische Phase mit 40 ml 5 %-iger Salzsäure extrahiert. Die vereinigten wäßrigen Phasen wurden zweimal mit 50 ml Ether gewaschen. Zur Freisetzung der Base wurde mit konzentrierter Natronlauge versetzt und mit Dichlormethan extrahiert. Auf diese Weise wurden 7,8 g Rohprodukt erhalten, das auf eine Säule, gefüllt mit Kieselgel, gegeben wurde. Die Elution mit Essigsäureethylester/Methanol = 1 : 1 ergab 4,89 g Base, aus der mit Trimethylchlorsilan/Wasser in 2-Butanon 4,6 g (44 % der Theorie) Hydrochlorid (12) mit einem Schmelzpunkt von 194-195 °C erhalten wurde.

2. Stufe:

(Z)-(RS)-[3-(3-Difluormethyl-phenyl)-2-methyl-pent-3-enyl]-dimethyl-amin, Hydrochlorid (11)

10 g (32 mmol) (2RS, 3RS)-3-(3-Difluormethyl-phenyl)-1-dimethylamino-2-methyl-pentan-3-ol, Hydrochlorid (12) aus Stufe 1 wurden in 150 ml konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/Ether versetzt. Nach Trocknung der organischen Phase und destillativer Entfernung des Lösungsmittels wurden 9,1 g (97 % der Theorie) Rohbase erhalten, die auf eine Säule, gefüllt mit Kieselgel, gegeben wurden. Die Elution mit Diisopropylether/ Methanol = 7 : 1 ergab 3,0 g Base, aus der mit Trimethylchlorsilan/Wasser in 2-Butanon 2,3 g (24 % der Theorie) Hydrochlorid (11) mit einem Schmelzpunkt von 160-161 °C erhalten wurden.

**Beispiel 12**

(Z)-(RS)-6-[1-(2-Dimethylamino-1-methyl-ethyl)-propenyl]-naphth-2-ol, Hydrochlorid (13)

Aus (1RS, 2RS)-6-(3-Dimethylamino-1-ethyl-1-hydroxy-2-methyl-propyl)-naphth-2-ol, Hydrochlorid, hergestellt gemäß Chirality 6, 389 (1994), wurde unter den in Beispiel 1 (2. Stufe) angegebenen Bedingungen das Hydrochlorid (13) in 39 %-iger Ausbeute mit einem Schmelzpunkt von 207 - 208 °C erhalten.

**Beispiel 13**

(E)-(RS)-[3-(3-Methoxy-phenyl)-2-methyl-hex-3-enyl]-dimethylamin, Hydrochlorid (14) und

(Z)-(RS)-[3-(3-Methoxy-phenyl)-2-methyl-hex-3-enyl]-dimethylamin, Hydrochlorid (15)

Ausgehend von (2RS)-3-Dimethylamino-1-(3-methoxy-phenyl)-2-methylpropan-1-on und 1-Brom-propan wurde unter den in Beispiel 1 (1. Stufe) angegebenen Bedingungen unter Verwendung von Ether als Lösungsmittel das (2RS, 3SR)-1-Dimethylamino-3-(3-methoxy-phenyl)-2-methyl-hexan-3-ol, Hydrochlorid (16) in einer Ausbeute von 81 % mit einem Schmelzpunkt von 131-132 °C erhalten. 30 g (0,1 mol) der Verbindung (16) wurden gemäß Beispiel 5 mit 450 ml konzentrierter Ameisensäure umgesetzt. Die auf diese Weise erhaltene Rohbase (28 g), bestehend aus einem (Z)/(E)-Isomerengemisch wurde auf eine Säule, gefüllt mit Kieselgel, gegeben. Die Elution mit Diisopropylether/ Methanol = 7 : 1 ergab 7 g Base der (E)-Verbindung (14) und 17 g Base der (Z)-Verbindung (15). Die Basen wurden mit Trimethylchlorsilan/ Wasser in 2-Butanon in die Hydrochloride überführt.

(14) Ausbeute: 5,9 g (21 % der Theorie)
Schmelzpunkt: 154 °C

(15) Ausbeute: 15,8 g (56 % der Theorie) Schmelzpunkt: 110-112 °C

**Beispiel 14**

(E)-(RS)-3-[1-(2-Dimethylamino-1-methyl-ethyl)-but-1-enyl]-phenol, Hydrochlorid (17)

Aus (14), hergestellt nach Beispiel 13, wurde mit Dichlormethan/Natronlauge die Base freigesetzt und nach dem Trocknen der Lösung Dichlormethan destillativ entfernt. Aus der so erhaltenen Base wurde unter den in Beispiel 2 angegebenen Bedingungen das Hydrochlorid (17) in einer Ausbeute von 86 % der Theorie und einem Schmelzpunkt von 214 °C erhalten.

**Beispiel 15**

(Z)-(RS)-3-[1-(2-Dimethylamino-1-methyl-ethyl)-but-1-enyl]-phenol, Hydrochlorid (18)

Aus (15), hergestellt nach Beispiel 13, wurde mit Dichlormethan/Natronlauge die Base freigesetzt und nach dem Trocknen der Lösung Dichlormethan destillativ entfernt. Aus der so erhaltenen Base wurde unter den in Beispiel 2 angegebenen Bedingungen das Hydrochlorid (18) in einer Ausbeute von 86 % der Theorie und einem Schmelzpunkt von 120-121 °C erhalten.

**Beispiel 16**

(RS)-[3-(3-Methoxy-phenyl)-2-propyl-but-3-enyl]-dimethylamin, Hydrochlorid (19)

Ausgehend von (RS)-2-Dimethylaminomethyl-1-(3-methoxy-phenyl)-pentan-1-on und Methyliodid wurde unter den in Beispiel 1 (1. Stufe) angegebenen Bedingungen unter Verwendung von Ether als Lösungsmittel (2RS, 3SR)-3-Dimethylaminomethyl-2-(3-methoxy-phenyl)-hexan-2-ol, Hydrochlorid (20) in einer Ausbeute von 76 % mit einem Schmelzpunkt von 137-138 °C erhalten. 30 g (0,1 mol) der Verbindung (20) wurden gemäß Beispiel 5 mit 300 ml konzentrierter Ameisensäure umgesetzt. Die erhaltene Rohbase wurde auf eine Säule, gefüllt mit Kieselgel, gegeben. Die Elution mit Diisopropylether/Methanol = 7 : 1 ergab 24 g Base, aus der mit Trimethylchlorsilan/Wasser in 2-Butanon 23,1 g (74 % der Theorie) Hydrochlorid (19) mit einem Schmelzpunkt von 120-121 °C erhalten wurden.

**Beispiel 17**

(RS)-3-[1-(2-Dimethylamino-1-methyl-ethyl]-vinyl]-phenol, Hydrochlorid (21)

1. Stufe:

(1RS, 2SR)-3-(3-Dimethylamino-1-hydroxy-1,2-dimethyl-propyl)-phenol, Hydrochlorid (22)

Ausgehend von (RS)-3-Dimethylamino-1-(3-methoxy-phenyl)-2-methylpropan-1-on und Methyliodid wurde unter den in Beispiel 1 (1. Stufe) angegebenen Bedingungen unter Verwendung von Ether als Lösungsmittel (2RS, 3SR)-4-Dimethylamino-2-(3-methoxy-phenyl)-3-methyl-butan-2-ol, Hydrochlorid (23) in einer Ausbeute von 46 % mit einem Schmelzpunkt von 178-179 °C erhalten. Aus (23) wurde mit Dichlormethan/Natronlauge die Base freigesetzt. Nach dem Trocknen der Lösung wurde Dichlormethan im Vakuum abdestilliert. 23,7 g (0,1 mol) der Base wurden gemäß Beispiel 2 mit Diisobutylaluminiumhydrid umgesetzt. Auf diese Weise konnten 18,5 g (71 % der Theorie) Hydrochlorid (22) mit einem Schmelzpunkt von 183-184 °C erhalten werden.

2. Stufe:

(RS)-3-[1-(2-Dimethylamino-1-methyl-ethyl]-vinyl]-phenol, Hydrochlorid (21)

10 g (37 mmol) Hydrochlorid (22) aus Stufe 1 wurden in 150 ml konzentrierter Ameisensäure gelöst und zwei Stunden unter Rückfluß erhitzt. Anschließend wurde die Ameisensäure im Wasserstrahlvakuum abdestilliert, der Rückstand in Eiswasser aufgenommen und mit Natronlauge/ Ether versetzt. Nach Trocknung der organischen Phase und destillativer Entfernung des Lösungsmittels wurden 9,1 g Rohbase erhalten, aus der mit konzentrierter Salzsäure in Aceton 7,5 g (83% der Theorie) Hydrochlorid (21) mit einem Schmelzpunkt von 228-230 °C erhalten wurden.

**Beispiel 18**

(RS)-3-[1-(2-Dimethylamino-1-methyl-ethyl)-2-methyl-propenyl]-phenol, Hydrochlorid (24)

1. Stufe:

(RS)-[3-(3-Methoxy-phenyl)-2,4-dimethyl-pent-3-enyl]-dimethylamin (25)

Ausgehend von (RS)-1-Dimethylamino-2,4-dimethyl-pentan-3-on und 1-Brom-3-methoxy-benzol wurde unter den in Beispiel 1 (1. Stufe) angegebenen Bedingungen (2RS, 3RS)-1-Dimethylamino-3-(3-methoxyphenyl)-2,4-dimethyl-pentan-3-ol, Hydrochlorid (26) in einer Ausbeute von 44 % mit einem Schmelzpunkt von 180-181 °C erhalten. 30 g (0,1 mol) der Verbindung (26) wurden gemäß Beispiel 5 mit 450 ml konzentrierter Ameisensäure umgesetzt. Die erhaltene Rohbase wurde auf eine Säule, gefüllt mit Kieselgel, gegeben. Die Elution mit Diisopropylether/Methanol = 7 1 ergab 19 g Base (77 % der Theorie) als hellgelbes viskoses Öl.

2. Stufe:

(RS)-3-[1-(2-Dimethylamino-1-methyl-ethyl)-2-methyl-propenyl]-phenol, Hydrochlorid (24)

Aus der nach Stufe 1 erhaltenen Base wurde unter den in Beispiel 2 angegebenen Bedingungen das Hydrochlorid (24) in einer Ausbeute von 84 % der Theorie und einem Schmelzpunkt von 176-177 °C erhalten.

**Pharmakologische Untersuchungen**

**Analgesieprüfung im Writhing-Test an der Maus**

Die analgetische Wirksamkeit wurde im Phenylchinon-induzierten Writhing an der Maus, modifiziert nach I. C. Hendershot, J. Forsaith, J. Pharmacol. Exp. Ther. 125, 237 - 240 (1959) untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige

gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Aus der dosisabhängigen Abnahme der Writhingreaktionen im Vergleich zu parallel untersuchten Tiergruppen, denen keine erfindungsgemäße Verbindungen appliziert wurde, wurden mittels Regressionsanalyse (Auswerteprogramm Martens EDV Service, Eckental) die $ED_{50}$-Werte der Writhingreaktion berechnet.

Alle untersuchten erfindungsgemäßen Verbindungen zeigten eine ausgeprägte analgetische Wirkung, die im Vergleich zu Tramadol verstärkt war.

Die Ergebnisse sind in der nachfolgenden Tabelle zusammengefaßt.

Tabelle:

| **Analgesieprüfung im Writhing-Test an der Maus** | |
|---|---|
| erfindungsgemäße Verbindung hergestellt nach Beispiel | $ED_{50}$ (mg/kg) |
| 2 | 1,37 |
| 3 (+)-Enantiomer | 2,25 |
| 3 (-)-Enantiomer | 0,98 |
| 4 | 1,64 |
| 12 | 0,97 |
| 13 | 2,96 |
| 15 | 1,33 |
| 18 | 2,07 |
| zum Vergleich: Tramadol | 3,68 |

## Patentansprüche

**1.** Dimethyl-(3-aryl-but-3-enyl)-aminverbindungen der Formel I,

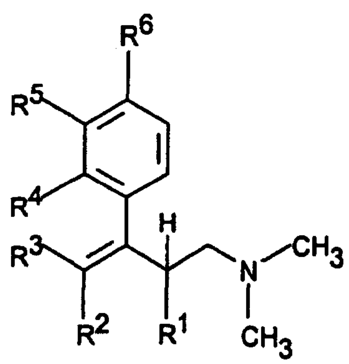

in der $R^1$ $C_{1-5}$-Alkyl ist und $R^2$ H oder $C_{1-5}$-Alkyl bedeutet,
$R^3$ H oder $C_{1-5}$-Alkyl bedeutet,
$R^4$ H, OH, $C_{1-4}$-Alkyl, O-$C_{1-4}$-Alkyl, O-Benzyl, $CF_3$, O-$CF_3$, Cl, F oder $OR^8$ bedeutet,
$R^5$ H, OH, $C_{1-4}$-Alkyl, O-$C_{1-4}$-Alkyl, O-Benzyl, $CHF_2$, $CF_3$, O-$CF_3$, Cl, F oder $OR^8$ darstellt und
$R^6$ H, OH, $C_{1-4}$-Alkyl, O-$C_{1-4}$-Alkyl, O-Benzyl, $CF_3$, O-$CF_3$, Cl, F oder $OR^8$ bedeutet,
mit der Maßgabe, daß zwei der Reste $R^4$, $R^5$ oder $R^6$ H sind, oder
$R^4$ und $R^5$ zusammen -CH=C($R^9$)-O- oder -CH=C($R^9$)-S- bedeuten, mit der Maßgabe, daß $R^6$ H ist, oder
$R^5$ und $R^6$ zusammen -CH=CH-C($OR^{10}$)=CH- bedeuten, mit der Maßgabe, daß $R^4$ H ist,
$R^8$ CO-$C_{1-5}$-Alkyl, PO(O-$C_{1-4}$-Alkyl$)_2$, CO-$C_6H_4$-$R^{11}$, CO(O-$C_{1-5}$-Alkyl), CO-$CHR^{12}$-$NHR^{13}$, CO-NH-$C_6H_3$-($R^{14})_2$ oder eine Pyridyl-, Thienyl-, Thiazoyl- oder Phenylgruppe bedeutet,
$R^9$ H oder $C_{1-4}$-Alkyl bedeutet,
$R^{10}$ H oder $C_{1-3}$-Alkyl bedeutet,
$R^{11}$ OC(O)-$C_{1-3}$-Alkyl in ortho-Stellung oder $CH_2$-N-($R^{15})_2$ in metaoder para-Stellung, wobei $R^{15}$ $C_{1-4}$-Alkyl oder beide Reste $R^{15}$ zusammen mit N den 4-Morpholino-Rest bilden, bedeutet,
$R^{12}$ und $R^{13}$ gleich oder verschieden sind und H, $C_{1-6}$-Alkyl oder $C_{3-8}$-Cycloalkyl oder $R^{12}$ und $R^{13}$ zusammen

$-(CH_2)_{3-8}-$ bedeuten,
$R^{14}$ H, OH, $C_{1-7}$-Alkyl, O-$C_{1-7}$-Alkyl, Phenyl, O-Aryl, $CF_3$, Cl oder F bedeutet, mit der Maßgabe, daß die beiden Reste $R^{14}$ gleich oder verschieden sind,
in Form ihrer Basen und/oder Salze von physiologisch verträglichen Säuren, als Enantiomere oder Racemate.

**2.** Dimethyl-(3-aryl-but-3-enyl)-aminverbindungen gemäß Anspruch 1, **dadurch gekennzeichnet, daß**
$R^1$ $C_{1-3}$-Alkyl ist und $R^2$ H oder $C_{1-3}$-Alkyl bedeutet,
$R^3$ H oder $C_{1-3}$-Alkyl bedeutet,
$R^4$ H, OH, $CF_3$, Cl, F oder $OR^8$ bedeutet,
$R^5$ H, OH, $C_{1-4}$-Alkyl, O-$C_{1-4}$-Alkyl, O-Benzyl, $CHF_2$, $CF_3$, Cl, F oder $OR^8$ darstellt und
$R^6$ H, OH, O-$C_{1-4}$-Alkyl, O-Benzyl, $CF_3$, Cl, F oder $OR^8$ bedeutet,
mit der Maßgabe, daß zwei der Reste $R^4$, $R^5$ oder $R^6$ H sind, oder
$R^4$ und $R^5$ zusammen $-CH=C(R^9)-O-$ oder $-CH=C(R^9)-S-$ bedeuten, mit der Maßgabe, daß $R^6$ H ist, oder
$R^5$ und $R^6$ zusammen $-CH=CH-C(OR^{10})=CH-$ bedeuten, mit der Maßgabe, daß $R^4$ H ist.

**3.** Dimethyl-(3-aryl-but-3-enyl)-aminverbindungen gemäß Anspruch 1 oder 2, **dadurch gekennzeichnet, daß**
$R^1$ $CH_3$ oder $C_3H_7$ und $R^2$ H, $CH_3$ oder $CH_2CH_3$ darstellen,
$R^3$ H, $CH_3$ oder $CH_2CH_3$ bedeutet,
$R^4$ H oder OH, $R^5$ H, OH, $OCH_3$, $CHF_2$ oder $OR^8$ und $R^6$ H, OH oder $CF_3$ bedeuten, mit der Maßgabe, daß zwei der Reste $R^4$, R5 oder $R^6$ H sind, oder
$R^4$ und $R^5$ zusammen $-CH=C(CH_3)-S-$ darstellen, mit der Maßgabe, daß $R^6$ H ist, oder
$R^5$ und $R^6$ zusammen $-CH=CH-C(OH)=CH-$ bedeuten, mit der Maßgabe, daß $R^4$ H ist, und
$R^8$ $CO-C_6H_4-R^{11}$ mit $R^{11}$ OC(O)-$C_{1-3}$-Alkyl in ortho-Stellung darstellt.

**4.** Dimethyl-(3-aryl-but-3-enyl)-aminverbindungen gemäß einem oder mehreren der Ansprüche 1 bis 3, **dadurch gekennzeichnet, daß**
$R^1$ $CH_3$ und $R^2$ H oder $CH_3$ bedeuten,
$R^3$ H oder $CH_3$ bedeutet,
$R^4$ H ist, $R^5$ OH oder $OR^8$ bedeutet, $R^6$ H ist, und $R^8$ $CO-C_6H_4-R^{11}$ mit $R^{11}$ $OC(O)-CH_3$ in ortho-Stellung bedeutet.

**5.** Verfahren zur Herstellung einer Dimethyl-(3-aryl-but-3-enyl)-aminverbindung der Formel I,

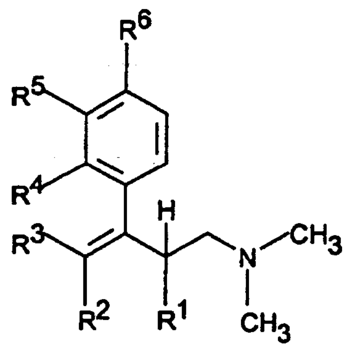

in der $R^1$ $C_{1-5}$-Alkyl ist und $R^2$ H oder $C_{1-5}$-Alkyl bedeutet,
$R^3$ H oder $C_{1-5}$-Alkyl bedeutet,
$R^4$ H, $C_{1-4}$-Alkyl, O-$C_{1-4}$-Alkyl, O-Benzyl, $CF_3$, O-$CF_3$, Cl oder F bedeutet,
$R^5$ H, $C_{1-4}$-Alkyl, O-$C_{1-4}$-Alkyl, O-Benzyl, $CHF_2$, $CF_3$, O-$CF_3$, Cl oder F darstellt und
$R^6$ H, $C_{1-4}$-Alkyl, O-$C_{1-4}$-Alkyl, O-Benzyl, $CF_3$, O-$CF_3$, Cl oder F bedeutet,
mit der Maßgabe, daß zwei der Reste $R^4$, $R^5$ oder $R^6$ H sind, oder
$R^4$ und $R^5$ zusammen $-CH=C(R^9)-O-$ oder $-CH=C(R^9)-S-$ bedeuten mit der Maßgabe, daß $R^6$ H ist, oder
$R^5$ und $R^6$ zusammen $-CH=CH-C(OR^{10})=CH-$ bedeuten, mit der Maßgabe, daß $R^4$ H ist,
$R^9$ H oder $C_{1-4}$-Alkyl und $R^{10}$ H oder $C_{1-3}$-Alkyl bedeuten, **dadurch gekennzeichnet, daß** man ein β-Dimethylaminoketon der Formel II

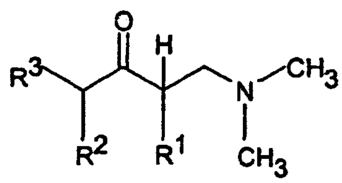

mit einer metallorganischen Verbindung der Formel III

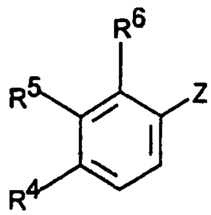

in der Z MgCl, MgBr, Mgl oder Li bedeutet, zu einem tertiären Alkohol der Formel IV

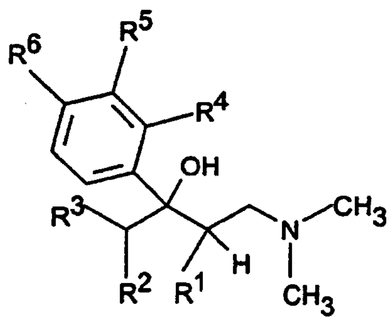

umsetzt, welcher anschließend zu einer Verbindung der Formel I dehydratisiert wird.

**6.** Verfahren zur Herstellung einer Dimethyl-(3-aryl-but-3-enyl)-aminverbindung der Formel I,

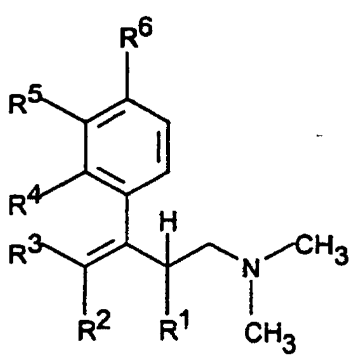

in der $R^1$ $C_{1-5}$-Alkyl ist und $R^2$ H oder $C_{1-5}$-Alkyl bedeutet,
$R^3$ H oder $C_{1-5}$-Alkyl bedeutet,
einer der Reste $R^4$, $R^5$ oder $R^6$ OH bedeutet und die anderen beiden Reste H sind,
**dadurch gekennzeichnet, daß** man eine Verbindung der Formel I, in der einer der Reste $R^4$, $R^5$ oder $R^6$ O-$CH_3$ bedeutet und die anderen beiden Reste H sind, mit Diisobutylaluminiumhydrid umsetzt oder eine Verbindung der

Formel I, in der einer der Reste $R^4$, $R^5$ oder R6 O-Benzyl bedeutet und die anderen beiden Reste H sind, reduktiv debenzyliert.

**7.** Arzneimittel enthaltend als pharmazeutischen Wirkstoff wenigstens eine Dimethyl-(3-aryl-but-3-enyl)-aminverbindung der Formel I gemäß Anspruch 1 in Form ihrer Base und/oder ihres Salzes einer physiologisch verträglichen Säure, als Enantiomer oder Racemat und gegebenfalls weitere Wirkstoffe und/oder Hilfsstoffe.

**8.** Arzneimittel nach Anspruch 7 zur Bekämpfung von Schmerz.

**9.** Verwendung wenigstens einer Dimethyl-(3-aryl-but-3-enyl)-aminverbindung der Formel I gemäß Anspruch 1 in Form ihrer Base und/oder ihres Salzes einer physiologisch verträglichen Säure, als Enantiomer oder Racemat zur Herstellung eines Arzneimittels zur Bekämpfung von Schmerzen.

## Claims

**1.** Dimethyl-(3-aryl-but-3-enyl)-amine compounds of formula I

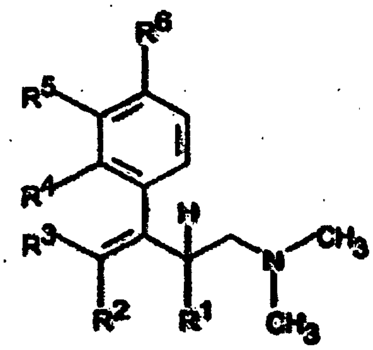

where $R^1$ is $C_{1-5}$ alkyl and $R^2$ denotes H or $C_{1-5}$ alkyl,
$R^3$ denotes H or Ci-s alkyl,
$R^4$ denotes H, OH, $C_{1-4}$ alkyl, O-$C_{1-4}$ alkyl, O-benzyl, $CF_3$, O-$CF_3$, Cl, F or $OR^8$,
$R^5$ represents H, OH, $C_{1-4}$ alkyl, O-$C_{1-4}$ alkyl, O-benzyl, $CHF_2$, $CF_3$, O-$CF_3$, Cl, F or $OR^8$, and
$R^6$ denotes H, OH, $C_{1-4}$ alkyl, O-$C_{1-4}$ alkyl, O-benzyl, $CF_3$, O-$CF_3$, Cl, F or $OR^8$,
with the proviso that two of the radicals $R^4$, $R^5$ or $R^6$ are H, or
$R^4$ and $R^5$ together denote -CH=C($R^9$)-O- or -CH=C($R^9$)-S-, with the proviso that $R^6$ is H, or
$R^5$ and $R^6$ together denote -CH=CH-C($OR^{10}$)=CH-, with the proviso that $R^4$ is H,
$R^8$ denotes CO-$C_{1-5}$ alkyl, PO(O-$C_{1-4}$ alkyl)$_2$, CO-$C_6H_4$-$R^{11}$, CO(O-$C_{1-5}$ alkyl), CO-CH$R^{12}$-NH$R^{13}$, CO-NH-$C_6H_3$-($R^{14}$)$_2$ or a pyridyl, thienyl, thiazoyl or phenyl group,
$R^9$ denotes H or $C_{1-4}$ alkyl,
$R^{10}$ denotes H or Ci-3 alkyl,
$R^{11}$ denotes OC(O)-$C_{1-3}$ alkyl in the ortho position or $CH_2$-N-($R^{15}$)$_2$ in the meta or para position, wherein $R^{15}$ denotes $C_{1-4}$ alkyl or both radicals $R^{15}$ form the 4-morpholino radical together with N,
$R^{12}$ and $R^{13}$ are the same or different and denote H, $C_{1-6}$ alkyl or $C_{3-8}$ cycloalkyl, or $R^{12}$ and $R^{13}$ together denote -$(CH_2)_{3-8}$-,
$R^{14}$ denotes H, OH, $C_{1-7}$ alkyl, O-$C_{1-7}$ alkyl, phenyl, O-aryl, $CF_3$, Cl or F, with the proviso that the two radicals $R^{14}$ are the same or different,
in the form of their bases and/or salts of physiologically compatible acids,
as enantiomers or racemates.

**2.** Dimethyl-(3-aryl-but-3-enyl)-amine compounds according to claim 1, **characterised in that**
$R^1$ is $C_{1-3}$ alkyl and $R^2$ denotes H or $C_{1-3}$ alkyl,
$R^3$ denotes H or Ci-3 alkyl,
$R^4$ denotes H, OH, $CF_3$, Cl, F or $OR^8$,
$R^5$ represents H, OH, $C_{1-4}$ alkyl, O-$C_{1-4}$ alkyl, O-benzyl, $CHF_2$, $CF_3$, Cl, F or $OR^8$, and

$R^6$ denotes H, OH, O-$C_{1-4}$ alkyl, O-benzyl, $CF_3$,Cl, F or $OR^8$,
with the proviso that two of the radicals $R^4$, $R^5$ or $R^6$ are H, or
$R^4$ and $R^5$ together denote -CH=C($R^9$)-O- or -CH=C($R^9$)-S-, with the proviso that $R^6$ is H, or
$R^5$ and $R^6$ together denote -CH=CH-C($OR^{10}$)=CH-, with the proviso that $R^4$ is H.

**3.** Dimethyl-(3-aryl-but-3-enyl)-amine compounds according to claim 1 or 2, **characterised in that** $R^1$ represents $CH_3$ or $C_3H_7$ and $R^2$ represents H, $CH_3$ or $CH_2CH_3$,
$R^3$ denotes H, $CH_3$ or $CH_2CH_3$,
$R^4$ denotes H or OH, $R^5$ denotes H, OH, $OCH_3$, $CHF_2$ or $OR^8$ and $R^6$ denotes H, OH or $CF_3$, with the proviso that two of the radicals $R^4$, $R^5$ or $R^6$ are H, or
$R^4$ and $R^5$ together represent -CH=C($CH_3$)-S-, with the proviso that $R^6$ is H, or
$R^5$ and $R^6$ together represent -CH=CH-C(OH)=CH-, with the proviso that $R^4$ is H, and
$R^8$ represents CO-$C_6H_4$-$R^{11}$, where $R^{11}$ represents OC(O)-$C_{1-3}$ alkyl in the ortho position.

**4.** Dimethyl-(3-aryl-but-3-enyl)-amine compounds according to one or more of claims 1 to 3, **characterised in that** $R^1$ denotes $CH_3$ and $R^2$ denotes H or $CH_3$,
$R^3$ denotes H or $CH_3$,
$R^4$ is H, $R^5$ denotes OH or $OR^8$, $R^6$ is H, and $R^8$ denotes CO-$C_6H_4$-$R^{11}$, where $R^{11}$ represents OC(O)-$CH_3$ in the ortho position.

**5.** A method of preparing a dimethyl-(3-aryl-but-3-enyl)-amine compound of formula I

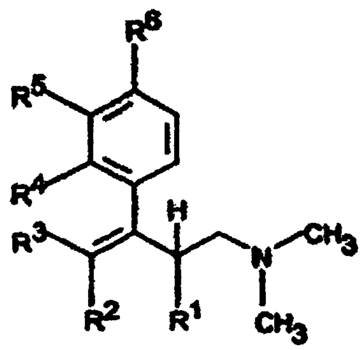

where $R^1$ is $C_{1-5}$ alkyl and $R^2$ denotes H or $C_{1-5}$ alkyl,
$R^3$ denotes H or $C_{1-5}$ alkyl,
$R^4$ denotes H, $C_{1-4}$ alkyl, O-$C_{1-4}$ alkyl, O-benzyl, $CF_3$, O-$CF_3$, Cl or F,
$R^5$ represents H, $C_{1-4}$ alkyl, O-$C_{1-4}$ alkyl, O-benzyl, $CHF_2$, $CF_3$, O-$CF_3$, Cl or F, and
$R^6$ denotes H, $C_{1-4}$ alkyl, O-$C_{1-4}$ alkyl, O-benzyl, $CF_3$, O-$CF_3$, Cl or F,
with the proviso that two of the radicals $R^4$, $R^5$ or $R^6$ are H, or
$R^4$ and $R^5$ together denote -CH=C($R^9$)-O- or -CH=C($R^9$)-S-, with the proviso that $R^6$ is H, or
$R^5$ and $R^6$ together denote -CH=CH-C($OR^{10}$)=CH-, with the proviso that $R^4$ is H,
$R^9$ denotes H or $C_{1-4}$ alkyl, and $R^{10}$ denotes H or $C_{1-3}$ alkyl, **characterised in that** a β-dimethylaminoketone of formula II

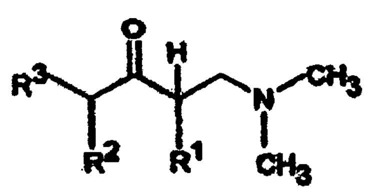

is reacted with an organometallic compound of formula III

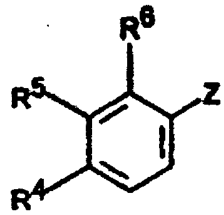

where Z denotes MgCl, MgBr, MgI or Li, to form a tertiary alcohol of formula IV

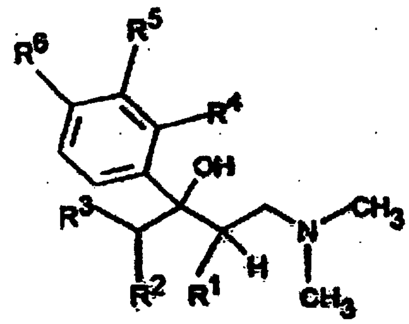

which is subsequently dehydrated to form a compound of formula I.

**6.** A method of preparing a dimethyl-(3-aryl-but-3-enyl)-amine compound of formula I

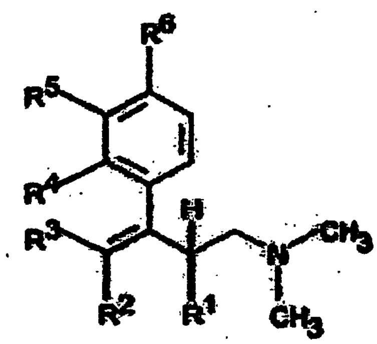

where $R^1$ is $C_{1-5}$ alkyl and $R^2$ denotes H or $C_{1-5}$ alkyl,
$R^3$ denotes H or $C_{1-5}$ alkyl,
one of the radicals $R^4$, $R^5$ or $R^6$ denotes OH and the other two radicals are H,
**characterised in that** a compound of formula I, in which one of the radicals $R^4$, $R^5$ or $R^6$ denotes $O\text{-}CH_3$ and the other two radicals are H, is reacted with diisobutylaluminium hydride, or a compound of formula I, in which one of the radicals $R^4$, $R^5$ or $R^6$ denotes O-benzyl and the other two radicals are H, is reductively debenzylated.

**7.** A drug containing, as a pharmaceutical active ingredient, at least one dimethyl-(3-aryl-but-3-enyl)-amine compound of formula I according to claim 1, in the form of its base and/or salt of a physiologically compatible acid, as an enantiomer or racemate, said drug optionally containing further active ingredients or adjuvants.

**8.** A drug according to claim 7 for controlling pain.

**9.** Use of at least one dimethyl-(3-aryl-but-3-enyl)-amine compound of formula I according to claim 1, in the form of its base and/or salt of a physiologically compatible acid, as an enantiomer or racemate, for producing a drug for controlling pain.

## Revendications

1. Composés de diméthyl-(3-aryl-but-3-ényl)-amines de formule I

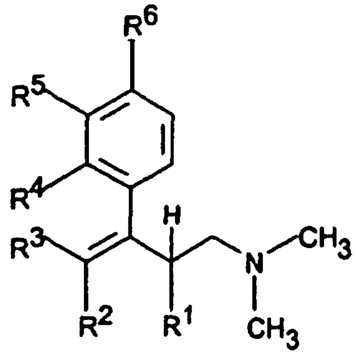

dans laquelle

$R^1$ est un reste alkyle en $C_1$ à $C_5$ et $R^2$ représente H ou un reste alkyle en $C_1$ à $C_5$,

$R^3$ représente H ou un reste alkyle en $C_1$ à $C_5$,

$R^4$ représente H, un groupe OH, un reste alkyle en $C_1$ à $C_4$, O-alkyle en $C_1$ à $C_4$, O-benzyle, $CF_3$, O-$CF_3$, Cl, F ou $OR^8$,

$R^5$ représente H, un groupe OH, un reste alkyle en $C_1$ à $C_4$, O-alkyle en $C_1$ à $C_4$, O-benzyle, $CHF_2$, $CF_3$, O-$CF_3$, Cl, F ou $OR^8$ et

$R^6$ représente H, un groupe OH, un reste alkyle en $C_1$ à $C_4$, O-alkyle en $C_1$ à $C_4$, O-benzyle, $CF_3$, $OCF_3$, Cl, F ou $OR^8$,

sous réserve que deux des restes $R^4$, $R^5$ ou $R^6$ représentent H, ou bien

$R^4$ et $R^5$ forment ensemble un groupement -CH=C($R^9$)-O- ou -CH=C($R^9$)-S-, sous réserve que $R^6$ représente H, ou bien

$R^5$ et $R^6$ forment ensemble un groupement -CH=CH-C($OR^{10}$)=CH- sous réserve que $R^4$ représente H,

$R^8$ est un reste CO-(alkyle en $C_1$ à $C_5$), PO(O-alkyle en $C_1$ à $C_4$)$_2$, CO-$C_6H_4$-$R^{11}$, CO(O-alkyle en $C_1$ à $C_5$), CO-CH$R^{12}$-NH$R^{13}$, CO-NH-$C_6H_3$-($R^{14}$)$_2$ ou un groupe pyridyle, thiényle, thiazoyle ou phényle,

$R^9$ représente H ou un reste alkyle en $C_1$ à $C_4$,

$R^{10}$ représente H ou un reste alkyle en $C_1$ à $C_3$,

$R^{11}$ est un groupement OC(O)-alkyle en $C_1$ à $C_3$ en position ortho ou un groupement $CH_2$-N-($R^{15}$)$_2$ en position méta ou para, $R^{15}$ désignant un reste alkyle en $C_1$ à $C_4$ ou bien les deux restes $R^{15}$ forment conjointement avec N le reste 4-morpholino,

$R^{12}$ et $R^{13}$ sont identiques ou différents et représentent H, un reste alkyle en $C_1$ à $C_6$ ou cycloalkyle en $C_3$ à $C_8$ ou bien $R^{12}$ et $R^{13}$ forment ensemble un groupement - $(CH_2)_{3-8}$-,

$R^{14}$ représente H, OH, un reste alkyle en $C_1$ à $C_7$, O-alkyle en $C_1$ à $C_7$, phényle, O-aryle, $CF_3$, Cl ou F, sous réserve que les deux restes $R^{14}$ soient identiques ou différents,

sous forme de leurs bases et/ou de leurs sels d'acides acceptables du point de vue physiologique, comme énantiomères ou racémates.

2. Composés de diméthyl-(3-aryl-but-3-ényl)-amines suivant la revendication 1, **caractérisés en ce que**

$R^1$ est un reste alkyle en $C_1$ à $C_3$ et $R^2$ représente H ou un reste alkyle en $C_1$ à $C_3$,

$R^3$ représente H ou un reste alkyle en $C_1$ à $C_3$,

$R^4$ représente H, OH, $CF_3$, Cl, F ou un reste $OR^8$,

$R^5$ représente H, OH, un reste alkyle en $C_1$ à $C_4$, O-alkyle en $C_1$ à $C_4$, O-benzyle, $CHF_2$, $CF_3$, Cl, F ou $OR^8$ et

$R^6$ représente H, OH, un reste O-alkyle en $C_1$ à $C_4$, O-benzyle, $CF_3$, Cl, F ou $OR^8$,

sous réserve que deux des restes $R^4$, $R^5$ ou $R^6$ représentent H, ou bien

$R^4$ et $R^5$ forment ensemble un groupement -CH=C($R^9$)-O- ou -CH=C($R^9$)-S-, sous réserve que $R^6$ représente H, ou bien

$R^5$ et $R^6$ forment ensemble un groupement -CH=CH-C($OR^{10}$)=CH-, sous réserve que $R^4$ représente H.

3. Composés de diméthyl-(3-aryl-but-3-ényl)-amines suivant la revendication 1 ou 2, **caractérisés en ce que**

$R^1$ représente un reste $CH_3$ ou $C_3H_7$ et $R^2$ représente H, un reste $CH_3$ ou $CH_2CH_3$,

$R^3$ représente H, un reste $CH_3$ ou $CH_2CH_3$,

$R^4$ représente H ou OH, $R^5$ représente H, OH, un reste $OCH_3$, $CHF_2$ ou $OR^8$ et $R^6$ représente H, OH ou un reste $CF_3$, sous réserve que deux des restes $R^4$, $R^5$ et $R^6$ représentent H, ou bien

$R^4$ et R $^5$ forment ensemble un groupement $-CH=C(CH_3)-S-$, sous réserve que $R^6$ représente H, ou bien

$R^5$ et $R^6$ forment ensemble un groupement -CH=CH-C(OH)=CH-, sous réserve que $R^4$ représente H, et

$R^8$ représente un groupement $CO-C_6H_4-R^{11}$ dans lequel $R^{11}$ est un reste OC(O)-alkyle en $C_1$ à $C_3$ en position ortho.

**4.** Composés de diméthyl-(3-aryl-but-3-ényl)-amines suivant une ou plusieurs des revendications 1 à 3, **caractérisés en ce que**

$R^1$ est un reste $CH_3$ et $R^2$ représente H ou un reste $CH_3$,

$R^3$ représente H ou un reste $CH_3$,

$R^4$ représente H, $R^5$ représente OH ou un reste $OR^8$, $R^6$ représente H et $R^8$ est un groupement $CO-C_6H_4-R^{11}$ dans lequel $R^{11}$ est un reste $OC(O)-CH_3$ en position ortho.

**5.** Procédé de production d'un composé de diméthyl-(3-aryl-but-3-ényl)-amine de formule I

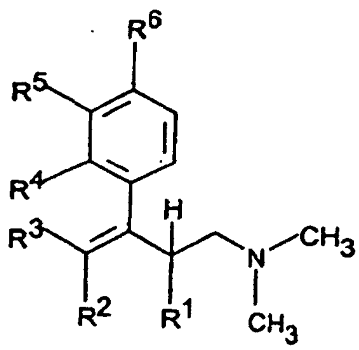

dans laquelle

$R^1$ est un reste alkyle en $C_1$ à $C_5$ et $R^2$ représente H ou un reste alkyle en $C_1$ à $C_5$,

$R^3$ représente H ou un reste alkyle en $C_1$ à $C_5$,

$R^4$ représente H, un reste alkyle en $C_1$ à $C_4$, O-alkyle en $C_1$ à $C_4$, O-benzyle, $CF_3$, $O-CF_3$, Cl ou F,

$R^5$ représente H, un reste alkyle en $C_1$ à $C_4$, O-alkyle en $C_1$ à $C_4$, O-benzyle, $CHF_2$, $CF_3$, $O-CF_3$, Cl ou F et

$R^6$ représente H, un reste alkyle en $C_1$ à $C_4$, O-alkyle en $C_1$ à $C_4$, O-benzyle, $CF_3$, $O-CF_3$, Cl ou F,

sous réserve que deux des restes $R^4$, $R^5$ ou $R^6$ représentent H, ou bien

$R^4$ et $R^5$ forment ensemble un groupement $-CH=C(R^9)-O-$ ou $-CH=C(R^9)-S-$, sous réserve que $R^6$ représente H, ou bien

$R^5$ et $R^6$ forment ensemble un groupement $-CH=CH-C(OR^{10})=CH-$, sous réserve que $R^4$ représente H,

$R^9$ représente H ou un reste alkyle en $C_1$ $C_4$ et $R^{10}$ représente H ou un reste alkyle en $C_1$ à $C_3$, **caractérisé en ce qu'**on fait réagir une β-diméthylaminocétone de formule II

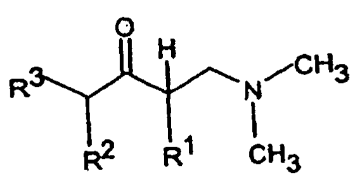

avec un composé organométallique de formule III

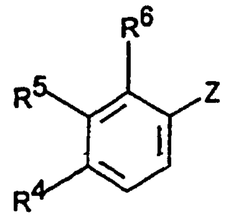

dans laquelle Z représente MgCl, MgBr, MgI ou Li, pour former un alcool tertiaire de formule IV

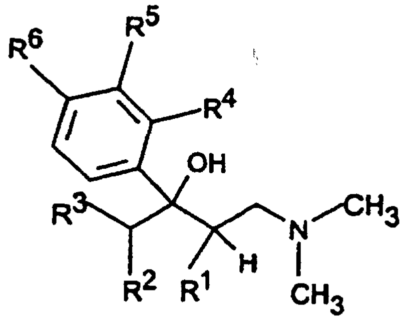

qu'on déshydrate ensuite pour obtenir un composé de formule I.

6. Procédé de production d'un composé de diméthyl-(3-aryl-but-3-ényl)-amine de formule I

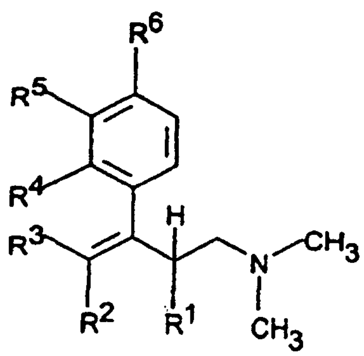

dans laquelle

$R^1$ est un reste alkyle en $C_1$ à $C_5$ et $R^2$ représente H ou un reste alkyle en $C_1$ à $C_5$,

$R^3$ représente H ou un reste alkyle en $C_1$ à $C_5$,

l'un des restes $R^4$, $R^5$ ou $R^6$ représente OH et les deux autres restes représentent H,

**caractérisé en ce qu'**on fait réagir un composé de formule I, dans laquelle l'un des restes $R^4$, $R^5$ ou $R^6$ désigne $O-CH_3$ et les deux autres restes représentent H, avec l'hydrure de diisobutylaluminium ou bien on débenzyle par voie de réduction un composé de formule I dans laquelle l'un des restes $R^4$, $R^4$ ou $R^6$ représente un reste O-benzyle et les deux autres restes représentent H.

7. Médicament contenant comme substance pharmaceutiquement active au moins un composé de diméthyl-(3-aryl-but-3-ényl)-amine de formule I suivant la revendication 1 sous forme de sa base et/ou de son sel d'un acide acceptable du point de vue physiologique comme énantiomère ou racémate et, le cas échéant, d'autres substances actives et/ou substances auxiliaires.

**8.** Médicament suivant la revendication 7, destiné à combattre la douleur.

**9.** Utilisation d'au moins un composé de diméthyl-(3-aryl-but-3-ényl)-amine de formule I suivant la revendication 1, sous forme de sa base et/ou de son sel d'un acide acceptable du point de vue physiologique comme énantiomère ou racémate pour la préparation d'un médicament destiné à combattre des douleurs.